# EUROPÄISCHE PATENTSCHRIFT

(11) **EP 3 094 292 B1**
(45) Veröffentlichungstag und Bekanntmachung des Hinweises auf die Patenterteilung: **02.08.2017**
(21) Anmeldenummer: 15728472.0
(22) Anmeldetag: 03.06.2015
(51) Int. Cl.: A61F 13/00

(54) **BANDAGE ZUR BEHANDLUNG VON LYMPHÖDEMEN**
BANDAGE FOR TREATING LYMPHEDEMAS
BANDE POUR LE TRAITEMENT DE LYMPHOEDÈMES

(30) Priorität: 06.06.2014 DE 102014108009
(43) Veröffentlichungstag der Anmeldung: 23.11.2016
(73) Patentinhaber: BSN medical GmbH, 20253 Hamburg (DE)
(72) Erfinder: SCHÜTZ, Patrick, 21075 Hamburg (DE)
(74) Vertreter: Uexküll & Stolberg
(86) Internationale Anmeldenummer: PCT/EP2015/062422
(87) Internationale Veröffentlichungsnummer: WO 2015/185652

(56) Entgegenhaltungen:
- WO-A2-2011/103527
- DE-A1-102005 007 016
- US-A1- 2003 104 746
- US-A1- 2014 121 627

## Beschreibung

Die vorliegende Erfindung betrifft eine elastische Bandage, die sich insbesondere zur Behandlung von Lymphstau, Lymphödemen und Elephantiasis eignet. Außerdem betrifft die Erfindung ein Verfahren zur Herstellung einer solchen Bandage.

Das Lymphödem ist eine chronische Erkrankung als Folge einer primären oder sekundären Schädigung des Lymphdrainagesystems, d.h. der Lymphkapillaren, Lymphkollektoren, Lymphknoten und/oder Lymphstämmen. Das insuffizient gewordene Lymphdrainagesystem ist nicht mehr in der Lage, die physiologischen lymphpflichtigen Lasten zu bewältigen. Dies hat einen Rückstau von Lymphflüssigkeit im Gewebe zur Folge, was zu einer Schwellung führt. Im weiteren Verlauf können pathologische Veränderungen auftreten, wie die Zunahme der Bindegewebszellen und der Bindegewebsfasern. Eine Entzündung des lymphatischen Systems, z.B. als Folge einer Infektionskrankheit, kann zu einer abnormen Vergrößerung von Körperteilen, meist Beinen, führen (sogenannte Elephantiasis). Dabei können auch häufig Überlappungen der betroffenen Hautregionen auftreten.

Üblicherweise werden zur Behandlung von Lymphödemen und Elephantiasis die betroffenen Arme oder Beine der Patienten mit Kompressionsbandagen umwickelt. Der äußere Druck unterstützt den Abtransport der Lymphflüssigkeit und damit den Abbau des Lymphstaus. Durch die Kompressionstherapie wird die Flüssigkeit im Interstitium verdrängt und die Gewebsflüssigkeit strömt verstärkt in die Lymphkapillaren. Außerdem wird durch die Kompressionswirkung der Lymphfluss in den noch funktionsfähigen Lymphgefäßen erhöht. Kompressionsbandagen bieten den Vorteil, dass sie bei abnehmender Schwellung, d.h. bei Reduktion der Umfänge, kontinuierlich den betroffenen Körperteilen angepasst werden können.

Gewöhnlich werden komprimierende Wechselbandagen als Teil von Sets angeboten, die einen Schlauchverband aus Baumwolle zum Schutz der Haut, Polstermaterialien zur gleichmäßigen Druckverteilung und textilelastische Binden zur Bewirkung des Kompressionsdrucks aufweisen. Neben Polstermaterialien werden Hautfalten und Vertiefungen der Haut regelmäßig mit Schaumstoffmaterialien ausgefüllt. Die Sets umfassen somit üblicherweise eine Vielzahl von verschiedenen Bandagen und Materialien.

Die WO 2011/103527 beschreibt einen Verband, der für die Kompressionstherapie geeignet ist und feuchtigkeitsabführende Eigenschaften aufweist. Der Verband umfasst eine elastische Schicht, die den notwendigen Kompressionsdruck ausüben kann und Feuchtigkeit von der Haut des Patienten in eine darüber liegende feuchtigkeitsabsorbierende Schicht leiten kann. So werden zur Entstauungstherapie die betroffenen Körperteile nach dem Anbringen einer Hautkontaktschicht mit einer elastischen, feuchtigkeitsleitenden Textilschicht umwickelt, die eine erste, zur Haut gewandte hydrophobe Oberfläche und eine zweite, von der Haut abgewandte hydrophile Oberfläche aufweist. Diese feuchtigkeitsleitende Textilschicht wird anschließend mit einer feuchtigkeitsabsorbierenden Schicht und danach vorzugsweise mit einer weiteren elastischen Verbandsschicht umwickelt.

Die US 2014/121627 A1 offenbart eine Kompressionsbandage, die eine untere elastische Lage, eine elastische Zwischenlage und eine obere elastische Lage umfasst, wobei die Bandage einen ersten Randbereich und einen gegenüberliegenden zweiten Randbereich aufweist, der mit dem ersten Randbereich gekoppelt werden kann, und die Bandage mit einer einzigen Wicklung an ein zu behandelndes Körperteil angebracht werden kann.

Die US 2003/104746 A1 beschreibt ein laminiertes Verbundmaterial, das zur Herstellung von z.B. Bandagen geeignet ist und bei dem eine elastische Kernschicht zwischen einer oberen und einer unteren Vliesschicht angeordnet ist.

Die DE 10 2005 007016 A1 offenbart eine Wundauflage, bei der eine obere und eine untere Lage mittels Abstandsfäden elastisch federnd beabstandet gehalten werden.

Nachteilig an bekannten Bandagen ist, dass zur Kompressionstherapie eine Vielzahl von verschieden Bandagen und Materialien um die betroffenen Körperregionen gewickelt werden müssen. Häufig ist auch das Feuchtigkeitsmanagement unzureichend, d.h. die Feuchtigkeit der umwickelten Hautregionen wird nur ungenügend abgeführt, so dass die Gefahr einer bakteriellen Hautinfektion oder einer Pilzinfektionen steigt. Der vorliegenden Erfindung hat somit die Aufgabe zugrunde gelegen, eine Bandage bereitzustellen, die zur Kompressionstherapie und insbesondere zur Behandlung von Lymphödemen und Elephantiasis geeignet ist, wobei die Bandage eine ausreichende Elastizität aufweist, um den nötigen Kompressionsdruck auszuüben, und gleichzeitig für eine Belüftung der umwickelten Hautregionen sorgt und Feuchtigkeit sowie selbst viskose und proteinreiche Flüssigkeiten, wie Lymphflüssigkeit, von der Haut wegführt, um so die Gefahr von Infektionen der Haut zu verringern. Des Weiteren wäre es wünschenswert, die Anzahl von verschiedenen Bandagen und Materialien, die um die betroffenen Körperteile gewickelt werden müssen, und somit die Anzahl an Wickelvorgängen zu verringern. Das heißt eine einzelne Bandage sollte möglichst viele der oben genannten Funktionen übernehmen. Außerdem sollte die Bandage einfach herzustellen sein und sich gut der Anatomie des Patienten anpassen, um leicht angelegt werden zu können.

Es hat sich nun herausgestellt, dass diese Aufgaben durch eine Bandage mit den Merkmalen des vorgelegten Hauptanspruchs gelöst werden. Gegenstand der Erfindung ist eine elastische Bandage, die
(a) eine untere elastische Lage,
(b) eine Kernschicht, die eine Vielzahl von mit den elastischen Lagen (a) und (c) verbundenen Abstandsfäden aufweist, und
(c) eine obere elastische Lage,
umfasst. Neben den Abstandsfäden weist die Kernschicht (b) ferner eine Vielzahl von Kernschichtgarnen auf, die sich im Wesentlichen parallel zu den Ebenen der elastischen Lagen (a) und (c) erstrecken.

Die erfindungsgemäße Bandage eignet sich als Kompressionsbandage zum Umwickeln von Körperteilen, wie Armen oder Beinen, um beispielsweise den Abtransport von Lymphflüssigkeit und damit den Abbau eines Lymphstaus zu bewirken. Bei der erfindungsgemäßen Bandage handelt es sich mithin vorzugsweise um ein längselastisches, dreidimensionales Flächengebilde in Streifenform und insbesondere um ein längselastisches, dreidimensionales Flächengebilde in Streifenform aus einem Verbund der Lage (a), der Schicht (b) und der Lage (c). Des Weiteren ist es bevorzugt, dass sich die Lagen (a) und (c) sowie die Kernschicht (b) in Draufsicht jeweils im Wesentlichen über die gesamte Fläche der Bandage erstrecken.

Die untere elastische Lage (a) und die obere elastische Lage (c) können gleich oder verschieden sein. Es ist bevorzugt, dass Lagen (a) und/oder (c) Textillagen, d.h. textile Flächengebilde, sind. Besonders bevorzugt sind die Lagen (a) und (c) unabhängig voneinander ausgewählt aus Gewirkebahnen und Gewebebahnen. Vorzugsweise sind die Lagen (a) und (c) unabhängig voneinander gewirkte, gewebte oder gestrickte Flächengebilde, die aus einem oder mehreren Faser- oder Fadentypen ausgewählt aus der Gruppe bestehend aus Polyestern, Polyamiden, Viskose, Baumwolle und deren Gemische und Kombinationen davon hergestellt sind.

Gemäß einer bevorzugten Ausführungsform können für die Erzeugung der Lagen (a) und (c) klassische Standardbindungen wie die klassische Trikotbindung verwendet werden. Besonders bevorzugt werden die Lagen (a) und/oder (c) durch ein Nähwirkverfahren, dem sog. Malimo-Verfahren, erzeugt.

Des Weiteren können die Lagen (a) und/oder (c) vorzugsweise auch unabhängig voneinander Filament- und/oder Endlosgarne, wie z.B. Monofilamente oder Multifilamente, umfassen. Durch die Verwendung von Filament- und/oder Endlosgarnen wird die Kapillarwirkung der Lagen (a) und (c) erhöht.

In einer weiteren besonders bevorzugten Ausführungsform weist das Gewirk oder Gestrick der unteren Lage (a), die bei der Verwendung der Bandage der Haut des Patienten zugewandt ist, eine größere Maschenweite als das Gewirk oder Gestrick der oberen Lage (c) auf, die der Haut des Patienten abgewandt ist. Dies hat den Vorteil, dass Feuchtigkeit oder Flüssigkeiten, wie Wund- oder Lymphflüssigkeit, von der Haut des Patienten leichter durch die Lage (a) in die Kernschicht (b) der Bandage eindringen kann und die Haut des Patienten somit trockner gehalten werden kann.

Der senkrechte Abstand der Lagen (a) und (c), und somit die Dicke der Kernschicht (b), beträgt im Ruhezustand der Bandage, d.h. ohne Druckbelastung, vorzugsweise 3 bis 12 mm, besonders bevorzugt 4 bis 7 mm.

Die Lagen (a) und (c) werden durch die Abstandsfäden in der Kernschicht (b) auf Distanz gehalten. Dies hat zur Folge, dass die Lagen (a) und (c) auch bei einer mechanischen Beanspruchung der Bandage durch z.B. festes Umwickeln von Körpergliedmaßen oder durch von außen auf die Bandage ausgeübten Druck, wie beispielsweise durch das Körpergewicht oder überlappende Hautfalten, nicht vollständig aufeinander liegen, sondern vielmehr stets eine Belüftung der Kernschicht und somit eine Belüftung der umwickelten Patientenhaut gewährleistet ist. Vorzugsweise bilden die Abstandsfäden zusammen mit den Lagen (a) und (c) ein sog. Abstandsgewirk wie z.B. ein kettengewirktes Abstandsgewirk.

Vorzugsweise beträgt die Zusammendrückbarkeit des Verbundes aus den Schichten (a), (b) und (c) mindestens etwa 2 kg/dm², besonders bevorzugt mindestens etwa 5 kg/dm². Dabei bezeichnet die Zusammendrückbarkeit gemäß DIN 53885 den Druck, der senkrecht zu den Lagen (a) und (c) ausgeübt wird und bei dem eine weitere Erhöhung des Druckes zu keiner weiteren Verringerung des Abstandes zwischen den Lagen (a) und (c) führt.

Die Druckfestigkeit und Scherstabilität des Verbundes aus den Lagen bzw. Schichten (a), (b) und (c) kann insbesondere durch die Anzahl der Abstandsfäden pro Fläche, deren Kreuzungswinkel, deren Dicke und das Material der Abstandsfäden eingestellt werden.

Gemäß einer Ausführungsform weist die Kernschicht (b) 500 bis 10.000, vorzugsweise 3.000 bis 8.000 Abstandsfäden pro m² auf, um eine besonders gute Druckfestigkeit zu erreichen.

In einer weiteren vorteilhaften Ausführungsform verlaufen die Abstandsfäden der Kernschicht in einem Winkel von zwischen 40° und 90°, vorzugsweise 75° und 90°, zu der Ebene der elastischen Lagen (a) und (c). Weiterhin ist es vorteilhaft, dass die Abstandsfäden kreuzförmig zwischen den Lagen (a) und (c) verlaufen. Damit ist eine gute Verschiebefestigkeit der Lagen (a) und (c) zueinander auch unter Druckbelastung und unter Einwirkung von Scherkräften gewährleistet.

Optional können zumindest ein Teil der Abstandfäden an ihren Kreuzungspunkten miteinander verbunden sein, z.B. durch Verschmelzen oder Verkleben. Dies führt zu einer weiteren Erhöhung der Festigkeit und Stabilität.

Es ist vorteilhaft, dass die Abstandsfäden Monofilamente umfassen. Vorzugsweise bestehen die Abstandsfäden im Wesentlichen aus Monofilamenten.

Des Weiteren ist es vorteilhaft, dass die Monofilamente Synthesefaserstoffe ausgewählt aus der Gruppe bestehend aus Polyestern, wie z.B. Polyethylentherephthalat oder Polybutylentherephthalat, Polyamiden, wie z.B. PA 6, PA 6.6, PA 6.12 oder PA 12, Polypropylenen und deren Gemischen oder Kombinationen umfassen.

Ferner weisen die Abstandsfäden vorzugsweise eine Feinheit von 5 bis 100 dtex, besonders bevorzugt 20 dtex bis 60 dtex auf.

Die Abstandsfäden sind vorzugsweise fest mit der unteren Lage (a) und der oberen Lage (c) verbunden. Besonders bevorzugt sind die Abstandsfäden z.B. durch Maschenbildung in die Lagen (a) und (c) eingebunden.

Neben den Abstandsfäden weist die Kernschicht (b) eine Vielzahl von Kernschichtgarnen auf, die sich im Wesentlichen parallel zu den Ebenen der elastischen Lagen (a) und (c) erstrecken und zum Transport und zur Speicherung von Feuchtigkeit dienen. Die Kernschichtgarne sind vorzugsweise hydrophil. Dies erhöht ihre Fähigkeit zum Transport und zur Speicherung von wässrigen Flüssigkeiten wie Lymphflüssigkeit.

Bevorzugt erstrecken sich die Kernschichtgarne vollständig in der Kernschicht zwischen den elastischen Lagen (a) und (c). Das heißt, dass in dieser Ausführungsform die Kernschichtgarne nicht in die Lagen (a) und (c) eingebunden, sondern lediglich in die Kernschicht (b) eingelegt sind. Dadurch lässt sich die Bandage besonders einfach herstellen.

In einer besonders bevorzugten Ausführungsform umfassen die Kernschichtgarne Filamentgarne, wie z.B. Multifilamente, und/oder Fasergarne.

Des Weiteren ist es bevorzugt, dass die Filamentgarne ausgewählt sind aus der Gruppe bestehend aus cellulosischen MultifilamentenDie cellulosischen Multifilamente sind insbesondere aus der Gruppe bestehend aus Viskose, Baumwolle, Lyocell und deren Gemische oder Kombinationen davon ausgewählt.

Die Fasergarne umfassen vorzugsweise Superabsorberfasern. Besonders bevorzugt umfassen die Superabsorberfasern Polymere auf Basis von Acrylsäure wie Co- oder Terpolymere und insbesondere quervernetzte Terpolymere umfassend Acrylsäure und Natriumacrylat und/oder Natriummethacrylat.

Besonders bevorzugt umfassen die Kernschichtgarne cellulosische Multifilamente, vorzugsweise Viskosefilamente, und Superabsorberfasern, vorzugsweise Co- oder Terpolymere auf Basis von Acrylsäure und Natriumacrylat und/oder Natriummethacrylat. Insbesondere ist es vorteilhaft, wenn das Gewichtsverhältnis von cellulosischen Multifilamenten zu Superabsorberfasern größer als 1:1 ist. Mithin umfassen die Kernschichtgarne vorzugsweise mehr als 50 Gew.-% cellulosische Multifilamente.

Mit den oben genannten Kernschichtgarnmaterialien oder Materialkombinationen kann eine Optimierung des Feuchtetransports erzielt werden.

Die Dochtwirkung und somit die Drainagewirkung der erfindungsgemäßen Bandage kann zweckmäßig mit einem Saughöhenprüfer gemäß DIN 53924 bestimmt werden. Dazu wird ein 5 cm breites und 20 cm langes Probenstück der Bandage senkrecht 1 cm tief in eine wässrige Testflüssigkeit getaucht. Nach 1 min wird die Saughöhe der Flüssigkeit gemessen, d.h. es wird gemessen, wie hoch die Testflüssigkeit in der Bandage gestiegen ist. Zweckmäßig wird bei dem Test eine gefärbte Flüssigkeit verwendet, um die Messung der Saughöhe zu erleichtern. Vorzugsweise beträgt die Saughöhe bei der erfindungsgemäßen Bandage mindestens 10 cm, besonders bevorzugt mindestens 12 cm.

Es hat sich herausgestellt, dass sich die erfindungsgemäße Bandage besonders gut zur Kompressionstherapie und insbesondere zur Behandlung von Lymphödemen und Elephantiasis eignet. Die elastischen Lagen (a) und (c) dienen dazu, dass mit der Bandage der gewünschte Kompressionsdruck eingestellt werden kann. Durch die abstandshaltende Kernschicht (b) bleibt die Bandage auch unter Druck atmungsaktiv. Dies sorgt für einen angenehmen Tragekomfort und fördert somit die Compliance bei den Patienten und damit den Therapieerfolg. Darüber hinaus sorgen die in die Kernschicht (b) eingelegten Filamente für ein aktives Feuchtigkeitsmanagement, indem sie Feuchtigkeit und selbst viskose und proteinreiche Flüssigkeiten wie Lymphflüssigkeit von der Lage (a) und somit von der Haut des Patienten wegführen. Dadurch wird die Gefahr von bakteriellen Infektionen und Pilzinfektionen beim Patienten verringert. Weiterhin ist mit der erfindungsgemäßen Bandage dessen Einsatz für die Kompressionstherapie im unmittelbaren Hautkontakt ohne Verwendung von Polstermaterial möglich, da die Kernschicht (b) durch die Abstandsfäden und insbesondere die in diese Schicht eingelegten Filamente selbst über gewisse Polstereigenschaften verfügt.

Optional kann die erfindungsgemäße Bandage eine oder mehrere zusätzliche Schichten oder Lagen aufweisen.

In einer Ausführungsform weist die Bandage zusätzlich eine Aufnahme- und Verteilerschicht (sog. Acquisition and Distribution Layer, ADL) auf, die dazu dient, Flüssigkeit aufzunehmen und zum Speicherbereich der Bandage in der Kernschicht weiterzuleiten. Eine derartige Aufnahme- und Verteilerschicht ist vorzugsweise oberhalb der oberen elastischen Lage (c), d.h. auf der von der Haut abgewandten Seiten der Bandage, angeordnet. Die Bereitstellung einer Aufnahme- und Verteilerschicht auf der Oberseite der Bandage führt zu einem besseren Flüssigkeitsmanagement. Insbesondere bewirkt eine Aufnahme- und Verteilerschicht auf der Oberseite der Bandage, dass im Bereich von Hautfalten und -überlappungen die Feuchtigkeit der von oben auf der Bandage aufliegenden Hautregion besser abgeführt werden kann.

Die Aufnahme- und Verteilerschicht wird vorzugsweise aus Fasergebilden ausgewählt aus der Gruppe bestehend aus Vliesstoffen, Tissue-Papieren und deren Kombinationen gebildet. Neben der oben beschriebenen Verbesserung des Flüssigkeitsmanagements führt die Verwendung einer solchen Aufnahme- und Verteilerschicht außerdem dazu, dass die Polsterfunktion der Bandage erhöht wird, so dass generell weniger separate Polstermaterialien verwendet werden müssen und die Anzahl der Wickelvorgänge reduziert werden kann.

Besonders bevorzugt umfasst die Aufnahme- und Verteilerschicht Vliesstoff, wie Spinnvlies mit hydrophilen Eigenschaften. Gemäß einer besonders vorteilhaften Ausführungsform ist der Vliesstoff ausgewählt aus der Gruppe bestehend aus Polyethylen, Polypropylen, Polyestern und deren Kombinationen.

Die Verwendung von Vliesstoffen und insbesondere den oben genannten Vliesstoffen hat den weiteren Vorteil, dass der Tragekomfort im Bereich von Hautfalten und -überlappungen erhöht wird, da der unmittelbare Hautkontakt mit Vliesstoffen von den Patienten üblicherweise als angenehm empfunden wird.

Das Flächengewicht des eingesetzten Vliesstoffes beträgt vorzugsweise 5 bis 100 g/m², besonders bevorzugt 10 bis 80 g/m².

Die Widerstandsfähigkeit gegen Flüssigkeitspenetration der Aufnahme- und Verteilerschicht beträgt vorzugsweise 0,3 bis 7 s, besonders bevorzugt etwa 2 bis 3 s. Dabei gibt die Widerstandsfähigkeit gegen Flüssigkeitspenetration die Zeit an, die 5 ml einer wässrigen Testflüssigkeit benötigen, um 5 mm der Aufnahme- und Verteilerschicht vollständig zu durchdringen. Vorzugsweise wird die Widerstandsfähigkeit gegen Flüssigkeitspenetration gemäß dem Standard WSP 70.3 z.B. mittels eines Lister AC Gerätes der Firma Lenzing Instruments gemessen.

Des Weiteren kann die erfindungsgemäße Bandage in einer Ausführungsform benachbart zu der unteren Lage (a) eine hydrophobe antimikrobielle Hautkontaktschicht aufweisen, die auf der der Kernschicht (b) abgewandten Oberfläche der unteren Lage (a) angeordnet ist. Insbesondere umfasst die Hautkontaktschicht ein Celluloseacetatgewebe, Viskosegewebe, Baumwollgewebe oder ein Mischgewebe, wobei als Mischgewebe vorzugsweise Schmelzklebfasern verwendet werden können. Besonders bevorzugt ist eine Schicht auf Basis von Celluloseacetatgewebe und Baumwollgewebe, insbesondere Celluloseacetatgewebe. Die Hydrophobierung der hydrophoben antimikrobiellen Hautkontaktschicht kann in einer Behandlung mit Dialkylcarbamoylchlorid (DACC) und/oder Alken-Keten-Dimer bestehen. In einer besonders bevorzugten Ausführungsform umfasst die Hautkontaktschicht Celluloseacetatgewebe, das mit DAAC, wie Dihexadecylcarbamoylchlorid oder Dioctadecyclcarbamoylchlorid, beschichtet ist. Die hydrophobe antimikrobielle Hautkontaktschicht weist vorzugsweise ein flächenspezifisches Gewicht von 40 bis 80 g/m², insbesondere 54 bis 66 g/m² und besonders bevorzugt etwa 60 g/m² auf. Des Weiteren beträgt die Dicke der Schicht vorzugsweise 0,2 bis 1,0 mm. Durch die Verwendung einer obigen Hautkontaktschicht kann die Gefahr von Infektionen der Haut der Patienten weiter verringert werden.

Das Flächengewicht der erfindungsgemäßen Bandage reicht vorzugsweise von 200 bis 600 g/m², insbesondere 250 bis 350 g/m².

Die Dehnbarkeit der erfindungsgemäßen Bandage beträgt vorzugsweise 30 bis 50%, gemessen gemäß DIN 53835, Teil 13, "Prüfung des zugelastischen Verhaltens". Dadurch wird erreicht, dass mit Hilfe der Bandage ein zum Abbau des Lymphstaus geeigneter Kompressionsdruck ausgeübt werden kann.

Schließlich beträgt die Dicke der erfindungsgemäßen Bandage vorzugsweise weniger als 10 mm, besonders bevorzugt 3 bis 7 mm. Es hat sich herausgestellt, dass bei höheren Dicken der Bandage ein Umwickeln der betroffenen Patientenkörperteile erschwert sein kann.

Vorzugsweise kann die erfindungsgemäße Bandage auf einer doppelbarrigen Raschelmaschine, wie z.B. HighDistance^{®}-Geräten der Firma Karl Mayer, hergestellt werden, die üblicherweise zur Herstellung von Abstandsgewirken verwendet werden. Die Kernschichtgarne werden dabei vorzugsweise mittig zwischen die Barren der Raschelmaschine zugeführt und somit in die erfindungsgemäße Bandage eingebracht.

Im Folgenden wird die vorliegende Erfindung anhand von lediglich bevorzugten Ausgestaltungen zeigenden Zeichnungen erläutert, wobei
- Fig. 1: einen schematischen Querschnitt durch eine erste bevorzugte Ausgestaltung der erfindungsgemäßen Bandage zeigt und
- Fig. 2: einen schematischen Querschnitt durch eine zweite bevorzugte Ausgestaltung der erfindungsgemäßen Bandage zeigt.

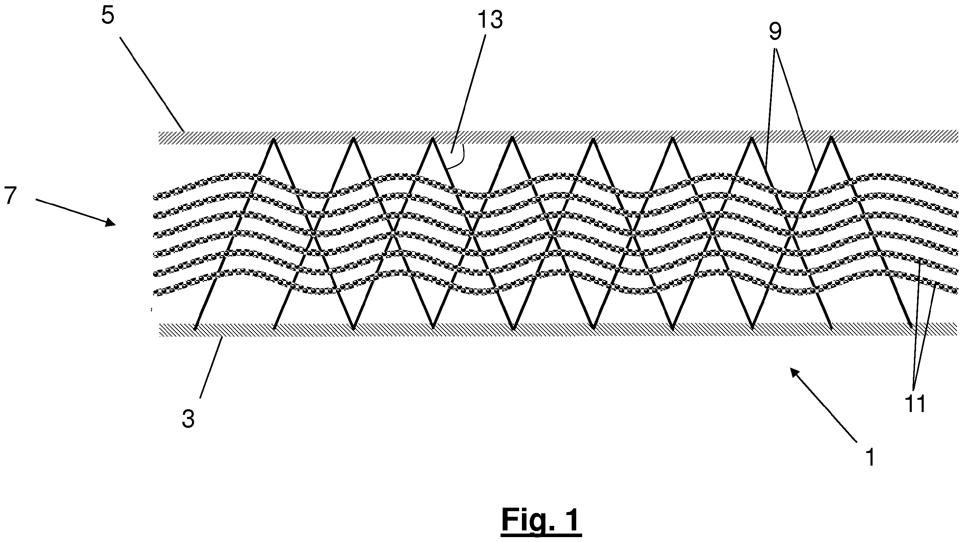

Figur 1 zeigt einen schematischen Querschnitt der erfindungsgemäßen Bandage. Die Bandage 1 weist als unterste Schicht eine untere elastische Lage 3 und als oberste Schicht eine obere elastische Lage 5 auf. Zwischen den Lagen 3 und 5 ist eine Kernschicht 7 angeordnet. Die Lagen 3 und 5 sowie die Kernschicht 7 erstrecken sich in dem gezeigten Querschnitt über die gesamte Länge der Bandage 1. Die Kernschicht 7 weist eine Vielzahl von Abstandsfäden 9 auf, die mit den Lagen 3 und 5 verbunden sind und diese auf Distanz halten, so dass die Lagen 3 und 5 auch bei einer mechanischen Beanspruchung der Bandage durch z.B. festes Umwickeln von Körpergliedmaßen oder durch von außen auf die Bandage ausgeübten Druck, wie beispielsweise durch das Körpergewicht oder überlappende Hautfalten, nicht vollständig aufeinander liegen, sondern vielmehr stets eine atmungsaktive Belüftung der Kernschicht und somit eine Belüftung der umwickelten Patientenhaut gewährleistet ist. Die Abstandsfäden 9 verlaufen kreuzförmig zwischen den Lagen 3 und 5 und bilden mit den Lagen 3 und 5 einen Winkel 13, der zwischen 40° und 90° liegt, wodurch selbst unter Druckbelastung und unter Einwirkung von Scherkräften eine gute Verschiebefestigkeit der Lagen 3 und 5 zueinander gewährleistet wird. Die Kernschicht 7 weist ferner eine Vielzahl von Kernschichtsgarnen 11 auf, die sich im Wesentlichen parallel zu den Ebenen der Lagen 3 und 5 und vollständig in der Kernschicht 7 erstrecken. Durch die Garne 11 ist die erfindungsgemäße Bandage 1 in der Lage, größere Mengen an Flüssigkeiten, insbesondere wässrigen Flüssigkeiten, wie viskoser Lymphflüssigkeit, von der Haut des Patienten abzutransportieren, wodurch das Flüssigkeitsmanagement der Bandage 1 im Vergleich zu herkömmlichen Bandagen erheblich verbessert wird.

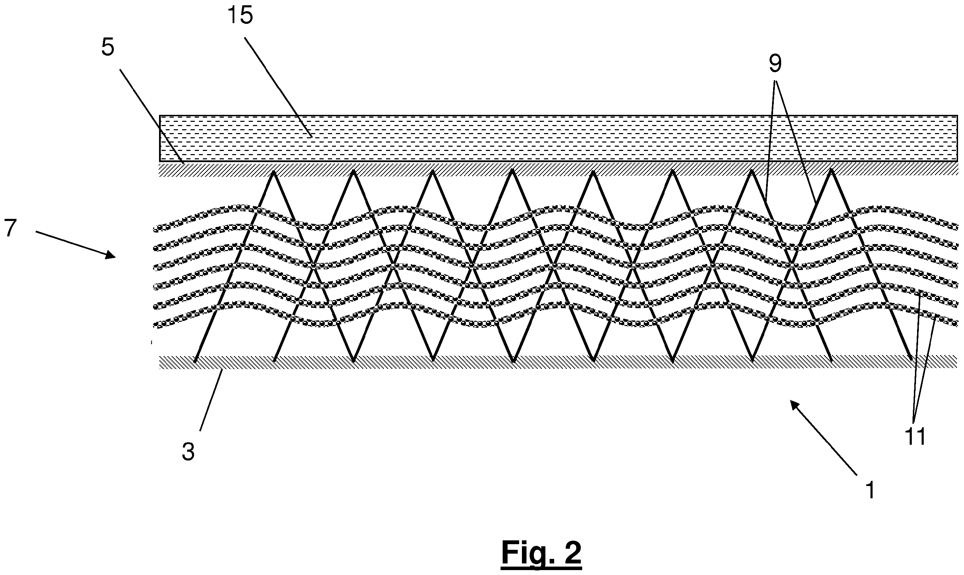

Im Vergleich zu der Bandage aus Figur 1 weist die in Figur 2 gezeigte Bandage 1 oberhalb der oberen Lage 5 eine zusätzliche Aufnahme- und Verteilerschicht 15 auf. Durch diese Schicht 15 wird die Kapazität der Bandage 1 zum Abtransport und Speichern von Flüssigkeit erhöht, so dass ein noch besseres Flüssigkeitsmanagement erzielt werden kann. Insbesondere bei der Anwendung der Bandage im Bereich von Hautfalten und Hautüberlappungen kann durch die Verwendung der Aufnahme- und Verteilerschicht 15 die Feuchtigkeit von oben auf der Bandage aufliegenden Hautregion besser abgeführt werden. Des Weiteren erhöht die Aufnahme- und Verteilerschicht 15 die Polsterfunktion der Bandage 1.

## Patentansprüche

1. Elastische Bandage (1) umfassend
(a) eine untere elastische Lage (3),
(b) eine Kernschicht (7), die eine Vielzahl von mit den elastischen Lagen (a) (3) und (c) (5) verbundenen Abstandsfäden (9) aufweist, und
(c) eine obere elastische Lage (5),
wobei die Kernschicht (b) (7) ferner eine Vielzahl von Kernschichtgarnen (11) aufweist, die sich im Wesentlichen parallel zu den Ebenen der elastischen Lagen (a) (3) und (c) (5) erstrecken.

2. Bandage nach Anspruch 1, bei der sich die Kernschichtgarne (11) vollständig in der Kernschicht (7) zwischen den elastischen Lagen (a) (3) und (c) (5) erstrecken.

3. Bandage nach Anspruch 1 oder 2, bei der die Kernschichtgarne (11) Filamentgarne und/oder Fasergarne umfassen.

4. Bandage nach Anspruch 3, bei der die Filamentgarne Multifilamente wie cellulosische Multifilamente umfassen.

5. Bandage nach Anspruch 4, bei der die cellulosischen Multifilamente ausgewählt sind aus der Gruppe bestehend aus Viskose, Baumwolle, Lyocell und deren Gemische oder Kombinationen davon.

6. Bandage nach Anspruch 3, bei der die Fasergarne Superabsorberfasern sind, die Polymere ausgewählt aus Co- und/oder Terpolymeren auf Basis von Acrylsäure und Natriumacrylat und/oder Natriummethacrylat und deren Gemische und Kombinationen enthalten.

7. Bandage nach einem der Ansprüche 4 bis 6, bei der die Kernschichtgarne (11) cellulosische Multifilamente, vorzugsweise Viskosefilamente, und Superabsorberfasern, vorzugsweise Co- und/oder Terpolymere auf Basis von Acrylsäure und Natriumacrylat und/oder Natriummethacrylat, umfassen, wobei das Gewichtsverhältnis von cellulosischen Multifilamenten zu Superabsorberfasern vorzugsweise mindestens 1:1 beträgt.

8. Bandage nach einem der vorhergehenden Ansprüche, bei der die Abstandsfäden (9) der Kernschicht (7) in einem Winkel (13) von zwischen 40° und 90°, vorzugsweise 75° und 90°, zu der Ebene der elastischen Lagen (a) (3) und (c) (5) verlaufen.

9. Bandage nach einem der vorhergehenden Ansprüche, bei der die Abstandsfäden (9) im Wesentlichen aus Monofilamenten bestehen, wobei die Monofilamente vorzugsweise Synthesefaserstoffe ausgewählt aus der Gruppe bestehend aus Polyestern, Polyamiden, Polypropylen und deren Gemischen und Kombinationen umfassen.

10. Bandage nach einem der vorhergehenden Ansprüche, bei der die Abstandsfäden (9) vorzugsweise eine Feinheit von 5 bis 100 dtex, besonders bevorzugt 20 dtex bis 60 dtex, aufweisen.

11. Bandage nach einem der vorhergehenden Ansprüche, bei der die untere Lage (a) (3) und die obere Lage (c) (5) gleich oder verschieden sein können und unabhängig voneinander gewirkte, gewebte oder gestrickte Flächengebilde sind, die aus einem oder mehreren Faser- oder Fadentypen ausgewählt aus der Gruppe bestehend aus Polyestern, Polyamiden, Viskose, Baumwolle und deren Gemischen oder Kombinationen hergestellt sind.

12. Bandage nach Anspruch 11, bei der das gewirkte, gewebte oder gestrickte Flächengebilde der unteren Lage (a) (3) eine größere Maschenweite als das gewirkte oder gestrickte Flächengebilde der oberen Lage (c) (5) aufweist.

13. Bandage nach einem der vorhergehenden Ansprüche, bei der der senkrechte Abstand der Lagen (a) (3) und (c) (5) 3 bis 12 mm, vorzugsweise 4 bis 7 mm, beträgt.

14. Bandage nach einem der vorhergehenden Ansprüche, die ferner eine Aufnahme- und Verteilerschicht (15) umfasst, wobei die Aufnahme- und Verteilerschicht (15) vorzugsweise auf der der Kernschicht (b) (7) abgewandten Oberfläche der oberen Lage (c) (5) angeordnet ist.

15. Bandage nach einem der vorhergehenden Ansprüche, die ferner eine hydrophobe antimikrobielle Hautkontaktschicht umfasst, die auf der der Kernschicht (b) (7) abgewandten Oberfläche der unteren Lage (a) (3) angeordnet ist.

## Claims

1. An elastic bandage (1) comprising
(a) a lower elastic layer (3),
(b) a core layer (7) having a plurality of spacer threads (9) connected to the elastic layers (a) (3) and (c) (5), and
(c) an upper elastic layer (5),
wherein the core layer (b) (7) further comprises a plurality of core layer yarns (11) which substantially extend parallel to the planes of the elastic layers (a) (3) and (c) (5).

2. A bandage according to claim 1, wherein the core layer yarns (11) completely extend in the core layer (7) between the elastic layers (a) (3) and (c) (5).

3. A bandage according to claims 1 or 2, wherein the core layer yarns (11) comprise filament yarns and/or fiber yarns.

4. A bandage according to claim 3, wherein the filament yarns comprise multifilaments as cellulosic multifilaments.

5. A bandage according to claim 4, wherein the cellulosic multifilaments are selected from the group consisting of viscose, cotton, lyocell and mixtures or combinations thereof.

6. A bandage according to claim 3, wherein the fiber yarns are super absorbent fibers comprising polymers selected from co- and/or terpolymers based on acrylic acid and sodium acrylate und/or sodium methacrylate and mixtures and combinations thereof.

7. A bandage according to any of the claims 4 to 6, wherein the core layer yarns (11) comprise cellulosic multifilaments, preferably viscose filaments, and super absorbent fibers, preferably co- and/or terpolymers based on acrylic acid and sodium acrylate and/or sodium methacrylate, wherein the weight ratio of cellulosic multifilaments to super absorbent fibers preferably is at least 1:1.

8. A bandage according to any of the proceeding claims, wherein the spacer threads (9) of the core layer (7) run in an angle (13) of between 40° and 90°, preferably 75° and 90°, to the plane of the elastic layers (a) (3) and (c) (5).

9. A bandage according to any of the proceeding claims, wherein the spacer threads (9) substantially consist of monofilaments, wherein the monofilaments preferably comprise synthetic fibers selected from the group consisting of polyesters, polyamides, polypropylene and mixtures and combinations thereof.

10. A bandage according to any of the proceeding claims, wherein the spacer threads (9) preferably have a yarn count of 5 to 100 dtex, most preferably 20 dtex to 60 dtex.

11. A bandage according to any of the proceeding claims, wherein the lower layer (a) (3) and the upper layer (c) (5) can be the same or different and independently from each other are knitted or woven textile fabrics which are produced from one or more fiber or thread types selected from the group consisting of polyesters, polyamides, viscose, cotton and mixtures and combinations thereof.

12. A bandage according to claim 11, wherein the knitted or woven textile fabric of the lower layer (a) (3) has a mesh size greater than the knitted textile fabric of the upper layer (c) (5).

13. A bandage according to any of the proceeding claims, wherein the perpendicular distance between the layers (a) (3) and (c) (5) is from 3 to 12 mm, preferably 4 to 7 mm.

14. A bandage according to any of the proceeding claims, further comprising an absorbing and distributing layer (15), wherein the absorbing and distributing layer (15) is preferably arranged on the surface of the upper layer (c) which faces away from the core layer (b) (7).

15. A bandage according to any of the proceeding claims, further comprising a hydrophobic antimicrobial skin contact layer which is arranged on the surface of the lower layer (a) (3) that faces away from the core layer (b) (7).

## Revendications

1. Bandage élastique (1), comprenant
(a) une couche élastique inférieure (3)
(b) une couche centrale (7), qui présente un grand nombre de fils d'écartement (9), reliés aux couches élastiques (a) (3) et (c) (5), et
(c) une couche élastique supérieure (5),
dans laquelle la couche centrale (b) (7) présente en outre un grand nombre de fils (11) de couche centrale, qui pour l'essentiel s'étendent parallèlement aux plans des couches élastiques (a) (3) et (c) (5).

2. Bandage selon la revendication 1, dans lequel les fils (11) de la couche centrale s'étendent complètement dans la couche centrale (7) entre les couches élastiques (a) (3) et (c) (5).

3. Bandage selon la revendication 1 ou 2, dans lequel les fils (11) de la couche centrale comprennent des fils continus et/ou des filés.

4. Bandage selon la revendication 3, dans lequel les fils continus comprennent des multifilaments, tels que des multifilaments cellulosiques.

5. Bandage selon la revendication 4, dans lequel les multifilaments cellulosiques sont choisis dans le groupe consistant en la viscose, le coton, le Lyocell et les mélanges ou combinaisons de ceux-ci.

6. Bandage selon la revendication 3, dans lequel les filés sont des fibres superabsorbantes, qui contiennent des polymères choisis parmi les co- et/ou les terpolymères à base d'acide acrylique et d'acrylate de sodium et/ou de méthacrylate de sodium et des mélanges et combinaisons de ceux-ci.

7. Bandage selon l'une des revendications 4 à 6, dans lequel les fils (11) de la couche centrale comprennent des multifilaments cellulosiques, de préférence des filaments de viscose et des fibres superabsorbantes, de préférence des co- et/ou des terpolymères à base d'acide acrylique et d'acrylate de sodium et/ou de méthacrylate de sodium, le rapport en poids entre les multifilaments cellulosiques et les fibres superabsorbantes étant de préférence d'au moins 1:1.

8. Bandage selon l'une des revendications précédentes, dans lequel les fils d'écartement (9) de la couche centrale (7) courent selon un angle (13) compris entre 40° et 90°, de préférence entre 75° et 90°, avec le plan des couches élastiques (a) (3) et (c) (5).

9. Bandage selon l'une des revendications précédentes, dans lequel les fils d'écartement (9) sont pour l'essentiel constitués de monofilaments, les monofilaments comprenant de préférence des matières fibreuses de synthèse choisies dans le groupe consistant en les polyesters, les polyamides, le polypropylène et les mélanges et combinaisons de ceux-ci.

10. Bandage selon l'une des revendications précédentes, dans lequel les fils d'écartement (9) présentent de préférence un titre de 5 à 100 dtex, d'une manière particulièrement préférée de 20 dtex à 60 dtex.

11. Bandage selon l'une des revendications précédentes, dans lequel la couche inférieure (a) (3) et la couche supérieure (c) (5) peuvent être identiques ou différentes et sont, indépendamment l'une de l'autre, des structures bidimensionnelles tricotées, tissées ou maillées, qui sont fabriquées à partir d'un ou plusieurs types de fibres ou de fils, choisis dans le groupe consistant en les polyesters, les polyamides, la viscose, le coton et les mélanges ou combinaisons de ceux-ci.

12. Bandage selon la revendication 11, dans lequel la structure bidimensionnelle tricotée, tissée ou maillée de la couche inférieure (a) (3) présente une ouverture de maille plus grande que la structure bidimensionnelle tricotée ou maillée de la couche supérieure (c) (5).

13. Bandage selon l'une des revendications précédentes, dans lequel la distance perpendiculaire des couches (a) (3) et (c) (5) est de 3 à 12 mm, de préférence de 4 à 7 mm.

14. Bandage selon l'une des revendications précédentes, qui comprend en outre une couche (15) de réception et de répartition, la couche (15) de réception et de répartition étant de préférence disposée sur la surface, opposée à la couche centrale (b) (7), de la couche supérieure (c) (5).

15. Bandage selon l'une des revendications précédentes, qui comprend en outre une couche hydrophobe antimicrobienne de contact avec la peau, qui est disposée sur la surface, opposée à la couche centrale (b) (7), de la couche inférieure (a) (3).
